# EUROPEAN PATENT SPECIFICATION

(11) **EP 4 299 092 B1**
(45) Date of publication and mention of the grant of the patent: **19.03.2025**
(21) Application number: 22305983.3
(22) Date of filing: 01.07.2022
(51) Int. Cl.: A61M 5/32

(54) **CAP FOR REMOVING A NEEDLE SHIELD ATTACHED TO A MEDICAL CONTAINER ARRANGED INSIDE A BODY OF AN AUTOINJECTOR**
KAPPE ZUM ENTFERNEN EINES NADELSCHUTZES, DER AN EINEM MEDIZINISCHEN BEHÄLTER BEFESTIGT IST, DER IM KÖRPER EINES AUTOINJEKTORS ANGEORDNET IST
CAPUCHON POUR RETIRER UNE PROTECTION D'AIGUILLE FIXÉE À UN RÉCIPIENT MÉDICAL DISPOSÉ À L'INTÉRIEUR D'UN CORPS D'UN INJECTEUR AUTOMATIQUE

(43) Date of publication of application: 03.01.2024
(73) Proprietor: Becton Dickinson France, 38800 Le Pont-de-Claix (FR)
(72) Inventor: MARITAN, Lionel, 38320 Bresson (FR); FIARD, Michael, 38320 Eybens (FR); DELOBELLE, Vincent, 38420 Domène (FR)
(74) Representative: Germain Maureau

(56) References cited:
- EP-A1- 2 878 321
- WO-A1-2017/102175
- US-A1- 2022 105 278

## Description

The present invention relates to a cap for an autoinjector, an autoinjector including said cap, and a method for assembling said autoinjector.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

Automatic injection devices are designed for automatic injection of a medical product into an injection site. Autoinjectors usually comprise a top body and a bottom body assembled to each other to form a housing. The bottom body is usually configured for receiving a medical container, such as a prefillable or prefilled syringe. The medical container has a barrel defining a reservoir for containing the medical product, the barrel having a distal end provided with an injection needle and an opened proximal end receiving a plunger rod for pushing a stopper. The injection needle is usually protected by a rigid needle shield removably secured to a distal tip of the medical container.

Autoinjectors also include a safety shield mechanism moving from an extended to a retracted position to respectively shield or unveil the needle and a power pack for automatically injecting the medical product into an injection site. The power pack is usually arranged inside the top body and includes a plunger rod for pushing a stopper inside the barrel of the medical container. An initially compressed spring is configured for moving the plunger in the distal direction. Locking means are provided for maintaining the plunger rod in an initial position in which the plunger rod is axially blocked despite the action of the compressed spring. A release member is further provided to release the plunger rod from the locking means and allow the spring to push the plunger rod in the distal direction to perform injection. A predetermined displacement of the safety shield towards the retracted position is required to allow the release member to unlock the locking means and release the plunger rod.

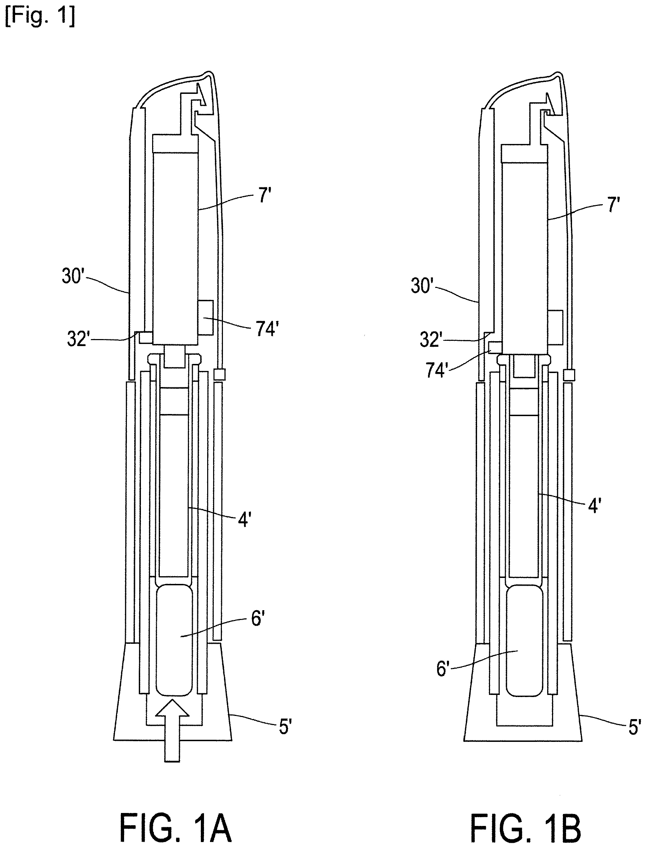

Assembly of the top body and the bottom body may be realized by any appropriate means such as snap-fitting or friction fit means. When fully assembled, a rattling noise may however be heard if a user shakes the autoinjector. This rattling noise is due to axial gaps that may exist between the powerpack and the medical container and between the power pack and the top body in the radial direction. As can be seen in Figures 1A-1B, the powerpack 7' may slightly move between a position (Figure 1A) in which the powerpack abuts against the top body 30' (and an axial clearance exists between the powerpack and the medical container 4') and another position (Figures 1B) in which the powerpack 7' abuts against the medical container 4' (and an axial clearance exists between the powerpack and the top body).

There is therefore a need for reducing these clearances and limiting the rattling noise.

The document US2022105278 discloses a cap provided at a distal end of an autoinjector. The cap includes a retainer for removing a needle shield. The retainer has radial protrusions for engaging an axial gap arranged between the needle shield and a distal shoulder of the syringe barrel. During assembly, it may happen that the needle shield is inserted too far inside the retainer. Thus, the radial protrusions outwardly deflect and rest against a lateral surface of the syringe barrel instead of engaging the axial gap. It is necessary to carry out a so-called "push back" step in which a the needle shield is moved back in the proximal direction. The push-back step enables the axial gap between the needle shield and the syringe barrel to move at the level of the radial protrusions so that the deformed radial protrusions move back inward in the axial gap and engage the needle shield. To that end, the known autoinjectors have an opened distal end providing access to the needle shield, thereby allowing to proximally push the needle shield. This opening at the distal end of the autoinjector is sometimes confusing for the end user who is generally not a skilled healthcare worker and who may believe that the injection needle will emerge from this central hole. It may happen that the end user forgets to remove the cap prior to injection.

There is therefore a need for a cap allowing to perform the push-back step and to avoid confusion for the end user.

An aspect of the invention is a cap for removing a needle shield attached to a medical container arranged inside a body of an autoinjector, the cap including:
a housing extending along a longitudinal axis A, the housing having a distal end and an opposite proximal end configured to be removably attached to the body of the autoinjector,
a retainer arranged inside the housing and axially movable with respect to said housing, the retainer including an inner cavity for receiving the needle shield, a clamping member configured to remove the needle shield from the medical container, an opened distal end configured to provide access to a distal end of the needle shield, and a proximal abutment surface configured to abut against the medical container,
a plug arranged at the distal end of the housing, the plug including a sealing portion configured for closing the distal end of the housing and at least one adjustment member arranged at a proximal side of the sealing portion for abutting against the retainer and move the retainer in the proximal direction.

As a result, the proximal abutment surface of the retainer pushes the medical container in the proximal direction. In turn, the medical container pushes the power pack against the body of the autoinjector. Thus, the axial clearances between the medical container, the power pack and the body of the autoinjector are suppressed or limited. The resulting rattling is also suppressed or limited.

The cap of the invention may further include some or all of the features below.

In an embodiment, the adjustment member includes a protrusion proximally extending from the proximal side of the sealing portion.

In an embodiment, the plug includes a single adjustment member.

The adjustment member may include two-diametrically opposite protrusions.

In an embodiment, the adjustment member includes a sloped surface configured to deform a distally extending resilient leg of the retainer.

In an embodiment, the adjustment member includes a resililently deformable element configured to exert a proximal pushing force on the retainer.

In an embodiment, the resiliently deformable member is in the form of a T-shaped blade.

In an embodiment, the plug further includes a push-back member for allowing to push the needle shield in a proximal direction.

In an embodiment, the push-back member is a through-hole extending through the sealing portion and configured to axially face a distal end of the needle shield.

In an embodiment, the push-back member includes a spring configured to push the needle shield in the proximal direction.

The adjustment member may include two proximal abutment surfaces and the push-back member is arranged between said two proximal abutment surfaces.

In an embodiment, the sealing portion is plate-shaped.

In an embodiment, the plug includes securing means for securing the plug to the housing. Thus, the plug is a separate component. The securing means are preferably configured to removably secure the plug to the housing.

In an alternative embodiment, the plug and the housing may be made of a single piece.

In an embodiment, the plug is made of a single piece.

Another aspect of the invention is an autoinjector including a cap having the aforementioned features.

Another aspect of the invention is a method for assembling said autoinjector, including the steps of :
(i) attaching the plug to the cap housing, said attachment causing the plug to push the retainer in the proximal direction so that the medical container abuts against a power pack of the autoinjector and the power pack abuts against the body of the autoinjector ;
(ii) pushing the needle shield back in the proximal direction so that the clamping member of the retainer engages an axial gap between the needle shield and a barrel of the medical container.

The above steps may be carried out in any chronological order. For instance, step (i) may occur before step (ii). In this case, the push-back member of the plug may be a through-hole providing acces to the distal end of the needle shield. In another instance, step (ii) may occur before step (i) : this may be the case when the plug is devoid of any push-back member. In another embodiment, steps (i) and (ii) occur at the same time. The plug automatically performs the push-back step (ii) when attached to the cap. This may be the case when the adjustment member of the plug includes a spring which pushes the needle shield.

The invention and the advantages arising therefrom will clearly emerge from the detailed description that is given below with reference to the appended drawings as follows :
- Figure 1A and 1B are schematic views illustrating the clerances of an autoinjector which is not part of the invention in, respectively, a first position in which the power pack abuts against the top body and a second position in which the power pack abuts against the syringe flange,
- Figure 2 is an exploded view of an autoinjector according to an embodiment of the invention,
- Figures 3A, 3B and 3C are perspective views and a cross section view of an outer part of a cap according to an embodiment of the invention,
- Figure 4 is a perspective view of a plug of a cap according to an embodiment of the invention,
- Figure 5A is a perspective view of a plug of a cap according to an embodiment of the invention,
- Figure 5B is a cross-section view of a distal end of an autoinjector equipped with the plug of Figure 5A,
- Figure 5C is a detail of Figure 5B,
- Figure 6A is a perspective view of a plug of a cap according to an embodiment of the invention,
- Figures 6B and 6C are cross-sectional views of an autoinjector equipped with the plug of Figure 6A,
- Figure 6D is a schematic view of an autoinjector equipped with the plug of Figure 5A,
- Figure 7 is a perspective view of a plug of a cap according to an embodiment of the invention,
- Figures 8A and 8B are perspective views of a retainer of a cap according to an embodiment of the invention,
- Figure 9 is a cross-section view of a cap housing and retainer assembled to the bottom body of an autoinjector according to an embodiment of the invention.

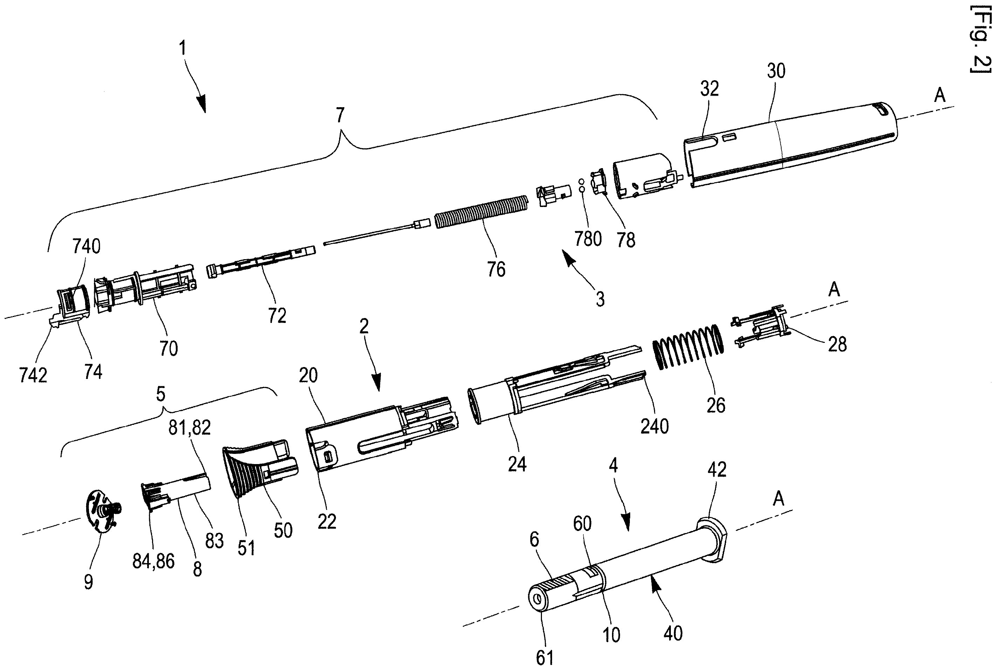

With reference to Figure 2 is shown an autoinjector 1 according to an embodiment of the invention. The autoinjector 1 is designed for automatic injection of a product into an injection site. The autoinjector 1 extends along a longitudinal axis A. The autoinjector 1 includes a lower sub-assembly 2 and a top sub-assembly 3 assembled to each other by any appropriate securing means such as, for instance, snap-fitting means.

The lower sub-assembly 2 includes a bottom body 20 for receiving a medical container 4, a cap 5 removably attached to a distal end 22 of the bottom body 20 and configured for removing a needle shield 6, a needle cover 24 axially movable along the longitudinal axis A with respect to the bottom body 20 between a first extended position (pre-use position) in which the needle cover 24 at least partially or completely shields an injection needle, and a retracted position (injection position) proximally located relative to said first extended position, in which the needle cover 24 moves inside the autoinjector 1 to trigger the injection. Movement of the needle cover 24 from the first extended position to the retracted position is caused by a distal end of the needle cover 24 being pressed against an injection site during use of the autoinjector 1. The needle cover 24 is further movable from the retracted position to a second extended position (safety position) in which the needle cover 24 moves back in the distal direction so as to safely shield the injection needle. The lower sub-assembly 2 may further include a safety spring 26 for urging the needle cover 24 in the distal direction towards the safety position and locking means for locking the needle cover 24 in said safety position. The locking means may include a locking element, such as an abutment ring 28 configured to be fixed to the medical container 4, having a proximally extending resilient leg that engages a two-way slot arranged through the needle cover 24.

The medical container 4 has a tubular barrel 40 defining a reservoir for containing a medical product to be injected. The barrel 40 has a distal end in the form of a distal shoulder and a longitudinally protruding tip provided with an injection needle. A needle shield 6 is removably attached to said distal end for protecting and sealing the injection needle. Opposite its distal end, the barrel 40 has an opened proximal end surrounded by a flange 42. The opened proximal end is configured to receive a plunger rod 72 for pushing a stopper arranged inside the barrel 40. The medical container 4 may be a prefilled or prefillable syringe.

The top sub-assembly 3 includes a top body 30 arranged for receiving a power pack 7. The power pack 7 is the unit that stores the energy and contain the features necessary to hold and release said energy so as to expel the medical product from the medical container. The power pack 7 may include a holder 70, a plunger rod 72 and an injection spring 76 configured to push the plunger rod 72 in the distal direction to perform injection. The plunger rod 72 is axially movable between an initial position, in which the plunger rod 72 is blocked axially in spite of the action of the retracted injection spring 76, and a final position, distally located with regard to said initial position, in which injection is completed. In the initial position, the plunger rod 72 may be axially away from the stopper, but in the final position the plunger rod 72 is engaged with the stopper and the injection spring 76 has extended in such a way that the plunger rod 72 has pushed the stopper to the distal end of the barrel 40 and the medical product is expelled from the reservoir. The power pack 7 may include blocking means for blocking the plunger rod 72 in the initial position, said blocking means including for instance an axially movable blocking ring 78 configured to maintain or release blocking balls 780 arranged for engaging radial cavities of the plunger rod 72.

The holder 70 is configured for triggering the injection operation. The holder 70 is axially movable inside the top body 30 between an initial position, in which the autoinjector 1 stays inactive, and a triggering position, proximally located with regard to said initial position, in which the holder 70 releases the plunger rod 72 so that the autoinjector 1 becomes activated. In the initial position, the holder 70 may stay axially away from the blocking ring 78, but in the triggering position the holder 70 may have pushed the blocking ring 78 in the proximal direction such that the blocking 780 balls are no longer blocked inside the cavities of the plunger rod 72 and can move outside said cavities in such a way that the plunger rod 72 is released. The proximal movement of the holder 70 from the initial to the triggering position may be caused by the needle cover 24 abutting against the holder 70 when the needle cover 24 moves towards the retracted position.

The power pack 7 may, or may not, includes a locker 74 arranged for preventing inadvertent movement of the holder 70 to the triggering position. The locker 74 may be in the form of a rotatable ring arranged around the holder 70. The locker 74 is axially movable between a an initial position, in which a proximal abutment surface 740 of the locker 74 axially faces a distal abutment surface which may be defined by an axial rib 32 of the top body 30, and an intermediate blocking position, proximally located with regard to said initial position, in which said proximal abutment surface 740 of the locker 74 abuts against the distal abutment surface of the top body 30 such that the locker 74 and the holder 70 (which axially abuts against the locker 74) are blocked in the proximal direction and cannot transition towards the triggering position. This prevents inadvertent activation of the autoinjector 1. However, the locker 74 is further rotationally movable around the longitudinal axis A between said intermediate blocking position and a release position, in which the proximal abutment surface 740 of the locker 74 is circumferentially shifted away from the distal abutment surface of the top body 30 such that the locker 74 does not any longer block the holder 70 and allows said holder 70 to move towards the triggering position. Translation and then rotation of the locker 74 are caused by a proximal end 240 of the needle cover 24 abutting against a cam portion 742 of the locker 74.

The cap 5 includes a housing 50, a retainer 8 arranged inside the housing 50, and a plug 9 arranged at a distal end 51 of the housing 50 for at least partially closing the opened distal end 51 of the housing 50.

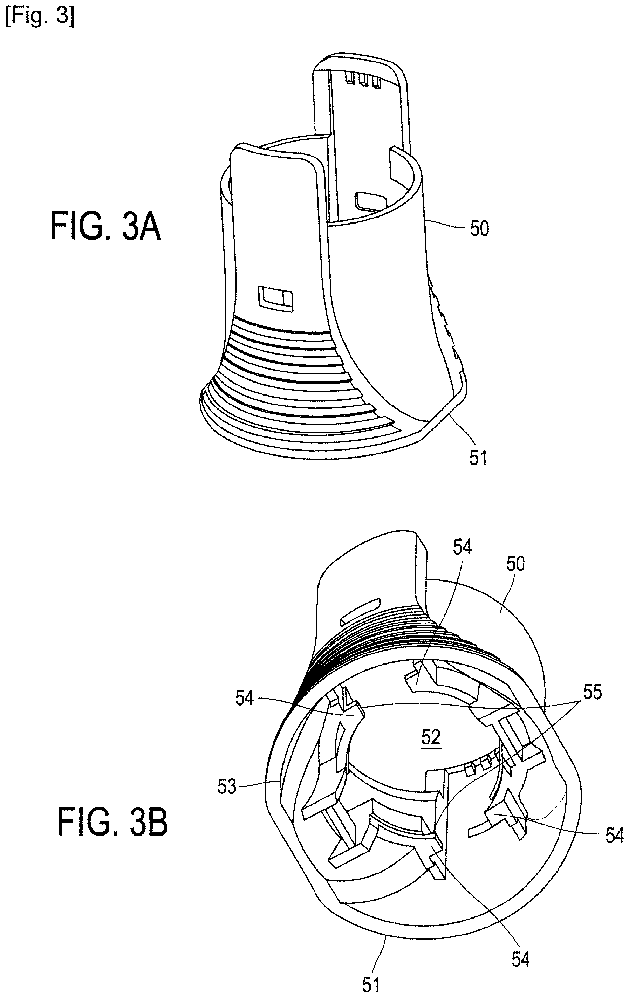

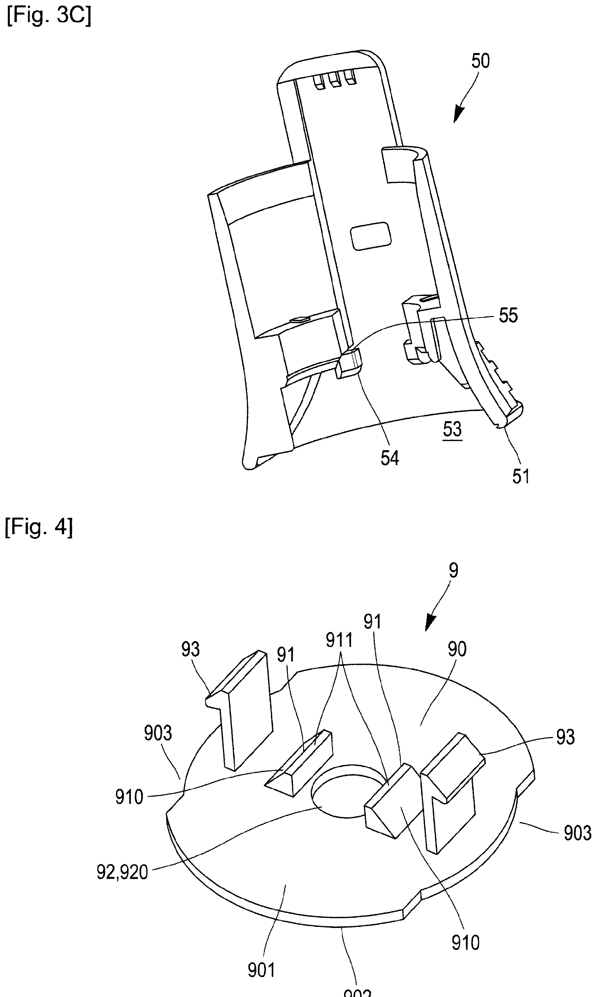

With reference to Figures 2 and 3A-3B, the cap housing 50 is arranged at a distal end 22 of the bottom body 20 and may include securing means for removable attachment to the bottom body 20, such as snap-fitting or friction fit means. The cap housing 50 may be tubular and may define an inner cavity 52 leading to a distal opening 53 allowing for insertion of the retainer 8 and/or the plug 9 inside the cap 5. The cap housing 50 may include two opposite axial shoulders 54, 55 extending inside the inner cavity 52 for limiting an axial movement of the retainer 8 with respect to the cap housing 50.

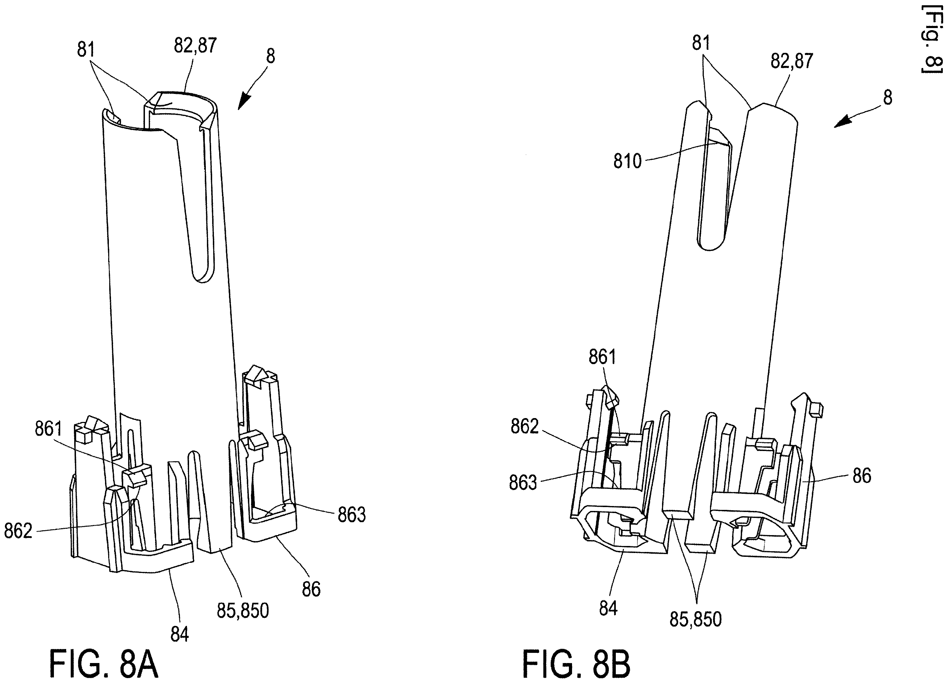

With reference to Figures 3B-3C and 8A-8B, the upper axial shoulder 55 may define a proximally oriented surface for abutting against a first abutment surface 862 of the retainer 8, thus preventing the retainer 8 from being pulled off the cap housing 50 in the distal direction. The lower axial shoulder 54 may define a distally oriented blocking surface for abutting against a second abutment surface 863 of the retainer 8, thus preventing disassembly of the retainer 8 and the cap housing 50. To that end, the retainer 8 may include a radial flange 86 defining the first and second abutment surfaces 862, 863 as visible in Figure 8A or 8B. The radial flange 86 may also include a chamfer 861 which may be arranged on the opposite side of the first abutment surface 862 for allowing the retainer 8 to pass beyond the lower axial shoulder 54 during assembly of the retainer 8 inside the cap housing 50.

The cap 5 is configured so that withdrawal of the cap housing 50 from the bottom body 20 of the autoinjector 1 causes withdrawal of the needle shield 6 from the medical container 4 by means of the retainer 8. More specifically, due to the abutment between the lower axial shoulder 54 and the second abutment surface 863, the cap housing 50 drives the retainer 8 in the distal direction. The clamping member 81 of the retainer 8 engages the proximal end 60 of the needle shield 6. The needle is accordingly withdrawn from the medical container 4. As visible in Figure 3A, the cap housing 50 may have a flared distal end 51 so that a user can firmly grasp the cap 5 and thus easily pull the cap 5 off the bottom body 20.

With reference to Figures 8A and 8B is shown the retainer 8. The retainer 8 is arranged within the cap housing 50 and may be in the form of a longitudinal sleeve defining an inner cavity for receiving the needle shield 6. The retainer 8 is configured to remove the needle shield 6 when the user removes the cap 5 from the autoinjector 1. To that end, the retainer 8 has a clamping member 81, such as a flexible hook arranged at a proximal end 82 of the retainer 8, and more specifically at the proximal end of distally extending resilient legs 83. The resilient legs 83 are configured to deform outwardly to allow insertion of the needle shield 6 within the retainer 8. At the end of the push-back step, the resilient legs 83 flex back towards the central longitudinal axis A to engage the axial gap 10 (Figure 2) between the needle shield 6 and the syringe barrel 40. The clamping member 81 may be provided with a distal stop 810 for abutting against a proximal end 60 of the needle shield 6.

The retainer 8 further has an opened distal end 84. The opening defined by said opened distal end 84 is configured for allowing the push-back step. The push-back step ends when the distal stop 810 abuts against the proximal end 60 of the needle shield 6. The distal end 84 of the retainer 8 is further configured to abut against the plug 9 so that the plug 9 can move the retainer 8 in the proximal direction when the plug 9 is assembled to the cap, as will be explained in further details below. More specifically, the distal end 84 of the retainer 8 may include at least one resilient leg 85 configured to abut against the plug 9.

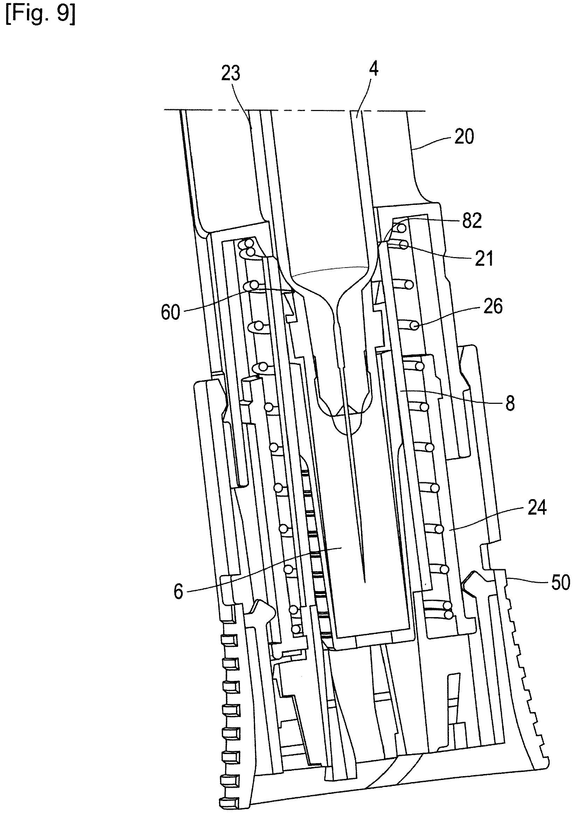

The retainer 8 may be axially movable with respect to the cap housing 50 between a first position, in which the retainer 8 abuts against the upper axial shoulder 55 of the cap housing 50, and a second position, proximally located with regard to the first position, in which the retainer 8 abuts either against the lower axial shoulder 54 or against the bottom body 20 (Figure 9). Before assembly of the cap 5 to the bottom body 20, the second position is defined by the retainer 8 abutting against the lower axial shoulder 54. When the cap 5 is assembled to the bottom body 20, the second position is defined by the proximal end 82 of the retainer 8 abutting against the bottom body 20. More specifically, as illustrated in Figure 9, the bottom body 20 may include a distal stop 21 for blocking the retainer 8 in the second position. The distal stop 21 may be arranged at a distal end of an inner cavity 23 which is configured for receiving the medical container 4. Thus, the retainer 8 cannot move further in the proximal direction. The retainer 8 is maintained in abutment against the bottom body 20 by the plug 9 whose anti-rattling member 91 pushes or abuts against the retainer 8.

The retainer 8 includes a proximal abutment surface 87 configured to abut against the medical container 4 when the retainer 8 moves in the proximal direction. The proximal abutment surface 87 may be defined by the proximal side of the hooks forming the clamping member 81, while the distal side of these hooks defines the distal stop 810. The proximal abutment surface 87 of the retainer 8 permits to push the medical container 4, and consequently the power pack 7, in the proximal direction so that the power pack 7 abuts against the top body 30 of the autoinjector 1. This limits or cancels the axial clearances and the rattling noise. Movement of the retainer 8 in the proximal direction is caused by the plug 9.

With reference to Figure 4 is shown a plug 9 according to an embodiment of the invention. The plug 9 is configured to delete the operating clearances between the power pack 7 and the medical container 4, thereby avoiding rattling noises. The plug 9 is arranged at the distal end 51 of the cap housing 50 and may include a sealing portion 90 for partially or completely closing the distal opening 53, an adjustment or anti-rattling member 91 for adjusting the axial position of the power pack 7 and the medical container 4 and thus cancelling clearances between the power pack 7 and the medical container 4, a push-back member 92 enabling to perform the push-back step, and securing means for securing the plug 9 to the rest of the cap 5. The plug 9 may be made of a single piece.

Still with reference to Figure 4, the sealing portion 90 may be in the form of a transversal plate extending orthogonal to the longitudinal axis A, such as for instance a plate-shaped disc, having a proximal side 901 and an opposite distal side 902. The sealing portion 90 and the distal opening 53 of the cap housing 50 may have complementary shapes, more specifically complementarily shaped outlines, so that the sealing portion 90 closes the inner cavity 52 of the cap 5, thereby avoiding confusion and misuse of the autoinjector 1 by the end user. The sealing portion 90 may include a notch 903 arranged at a lateral edge for properly positioning the plug 9 relative to the cap housing 50 during assembly of the plug 9 within the cap housing 50. The securing means for securing the plug 9 to the housing 50 may include snap-fitting means, such as two or more diametrically opposite flexible hooks 93 axially extending from the proximal side 901 of the transversal plate and configured for engaging complementary features of the cap housing 50.

The anti-rattling member 91 is configured to suppress the axial clearance between the power pack 7 and the medical container 4, thus cancelling the rattling noise that is due to said axial clearance. To that end, the anti-rattling member 91 is arranged at a proximal side 901 of the plug 9 for abutting against the retainer 8. The anti-rattling member 91 is intended to push the retainer 8 in the proximal direction when the plug 9 is secured to the cap housing 50. In turn, this causes the medical container 4 to move together with the retainer 8 in the proximal direction. Thus, the medical container 4 abuts against the power pack 7 and pushes the power pack 7 in abutment against the top body 30. The resilient legs 83 of the retainer 8 may then slightly deform in a radial outward direction until the proximal end 82 of retainer 8 abuts against the distal stop 21 of the bottom body 20 ; the clamping member 81 however remains engaged in the axial gap 10 and still faces the proximal end 60 of the needle shield 6. The anti-rattling member 91 thus axially maintains the medical container 4 against the power pack 7 and the power pack 7 against the top body 30 (as schematically illustrated in Figure 1A). The axial clearance and the consequent rattling noise are suppressed.

In the embodiment of Figure 4, the anti-rattling member 91 is in the form of a protrusion such as a tranversal rib which may be orthogonal to the longitudinal axis A and which is arranged on the proximal side 901 of the plug 9. The protrusion has a proximal abutment surface 910 which may be in the form of a sloped surface inclined with regard to the longitudinal axis A and configured to abut against the resilient legs 85 of the retainer 8 to outwardly deflect said resilsient legs 85. Deformation of the resilient legs 85 and their abutment againts the sloped surface 910 causes the retainer 8 to be pushed in the proximal direction. The plug 9 may include one, two or more anti-rattling members 91 which may be arranged diametrically opposite to each other. The anti-rattling members 91 are arranged at a distance from the lateral edge of the sealing portion 90. As illustrated in Figure 4, the anti-rattling members 91 may have a proximal end 911 which may define a flat transversal surface orthogonal to the longitudinal axis A.

The plug 9 may advantageously include a push-back member 92 for allowing to perform the push-back step, i.e. to push the needle shield 6 in the proximal direction until the distal stop 810 of the retainer 8 engages the axial gap 10 between the needle shield 6 and the syringe barrel 40 and thus faces the proximal end 60 of the needle shield 6. In the embodiment of Figure 4, the push-back member 92 includes a through-hole 920 which may be arranged at the center of the transversal plate forming the sealing portion 90 and which is configured to face the distal end 61 of the needle shield 6. The through-hole 920 is shaped to allow a machine to extend through the plug 9 so that the machine can push the retainer 8 in the proximal direction. The through-hole 920 may be arranged between the sloped surfaces 910 of the anti-rattling members 91. The anti-rattling members 91 may be arranged at a distance from the through-hole 920.

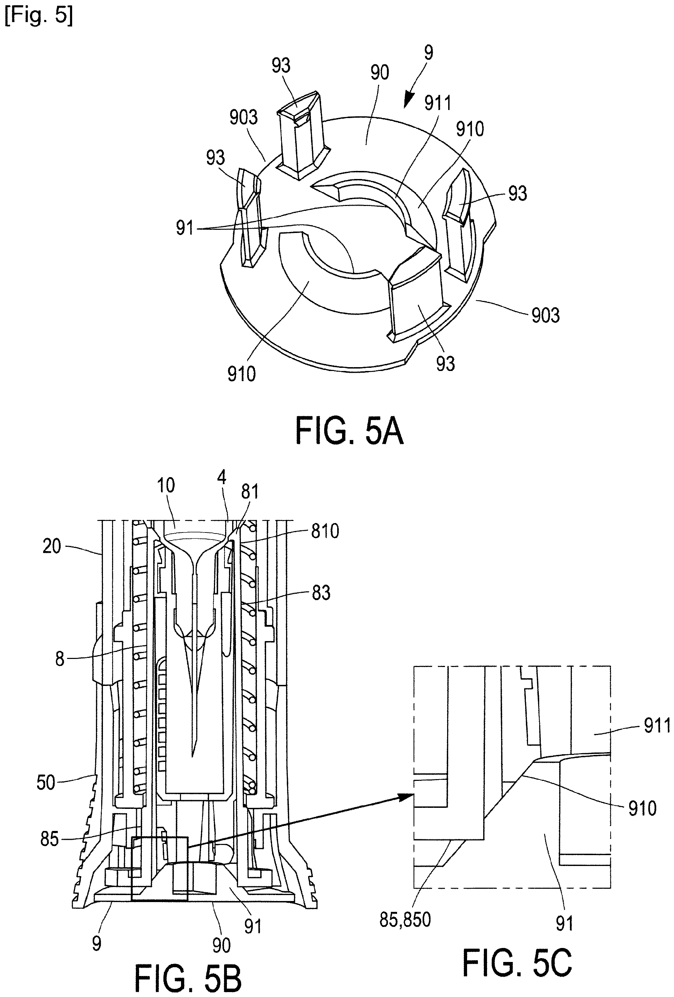

In the embodiment of Figures 5A-5C, the plug 9 is similar to the plug 9 of Figure 4 except that the securing means for securing the plug 9 to the housing 50 include four diametrically opposite flexible hooks 93 axially extending from the proximal side 901 of the transversal plate of the sealing portion 90 and configured for engaging complementary features of the cap housing 50. Besides, the anti-rattling member 91 is in the form of a circumferential rib extending around the central longitudinal axis A. The anti-rattling member 91 still has a proximal abutment surface in the form of a sloped surface 910 which is inclined with regard to the longitudinal axis A and which is configured to abut against the resilient legs 85 of the retainer 8 to outwardly deflect said resilsient legs. Deformation of the resilient legs 85 and their abutment againts the sloped surface 910 causes the retainer 8 to be pushed in the proximal direction. The plug 9 may include one, two or more anti-rattling members 91 which may be arranged diametrically opposite to each other. The anti-rattling members 91 are arranged at a distance from the lateral edge. The plug 9 of Figures 5A-5C does not include a push-back member 92 for allowing to perform the push-back step. The push-back step accordingly needs to be performed before assembly of the plug 9 within the cap housing 50.

Figure 5B and Figure 5C show the interference between the plug 9 and the retainer 8. As can be seen in these Figures, the anti-rattling member 91 of the plug 9 abuts against the distal end 850 of the resilient legs 85 of the retainer 8, thereby causing deformation of the resilient legs 85. The elastic deformation of the resilient legs 85 suppresses the clearances between the components and thus cancels the rattling noise.

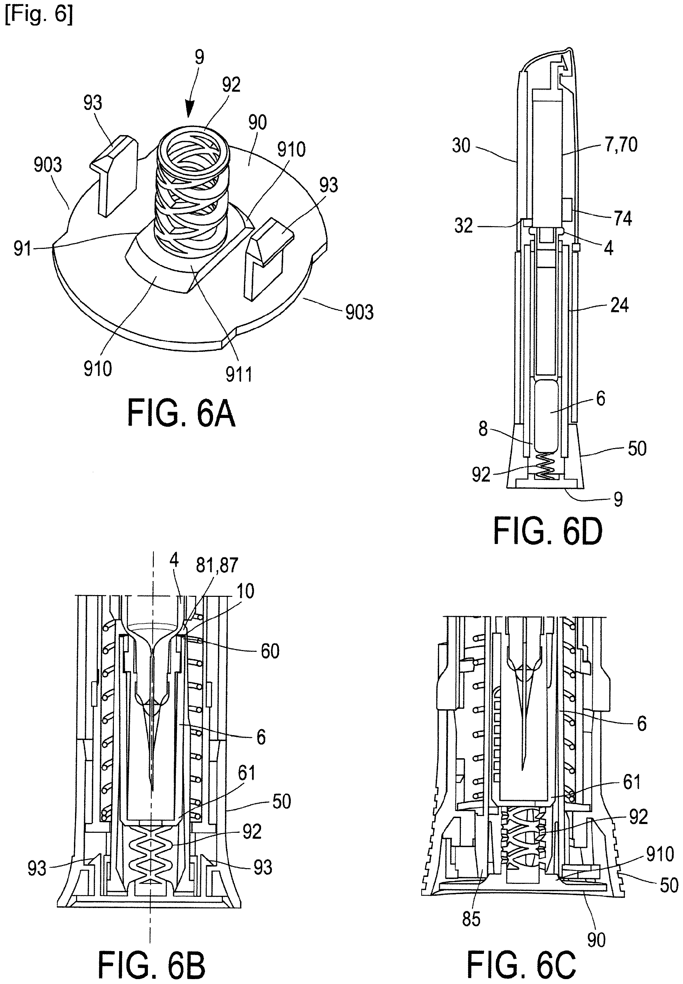

With reference to the embodiment illustrated in Figures 6A-6D, the plug 9 includes a single anti-rattling member 91. The anti-rattling member 91 has two diametrically opposite sloped surfaces 910 which are inclined with regard to the longitudinal axis A and which are configured to abut against the resilient legs 85 of the retainer 8 to outwardly deflect said resilsient legs 85. Deformation of the resilient legs 85 and their abutment againts the sloped surface 910 causes the retainer 8 to be pushed in the proximal direction. As illustrated in Figure 6A, the single anti-rattling member 91may have a proximal end 911 which may define a flat transversal surface orthogonal to the longitudinal axis A. Furthermore, the plug 9 advantageously includes a push-back member 92 for allowing to perform the push-back step, i.e. to push the needle shield 6 in the proximal direction until the distal stop 810 of the retainer 8 engages the axial gap 10 between the needle shield 6 and the syringe barrel 40 and thus axially faces the proximal end 60 of the needle shield 6. In the embodiment of Figure 6A, the push-back member 92 is formed by a resilient element, such as a spring, which may be arranged at the center of the anti-rattling member 91 or the center of the transversal plate forming the sealing portion 90 and which is configured to face the distal end 61 of the needle shield 6. The resilient element 92 extends axially and is configured to automatically perform the push-back step, i.e. without need for a machine and an additional time-consuming step to push the retainer 8 in the proximal direction. The resilient element 92 may be arranged between the sloped surfaces 910. The resilient element 92 is preferably integral with the plug 9 which may thus be made of a single piece.

Figures 6B and 6C illustrate the plug 9 mounted within the cap housing 50 and the resilient element 92 automatically pushing against the distal end 61 of the needle shield 6 so that the needle shield 6 and the medical container 4 move axially with regard to the retainer 8 until the distal stop 810 of the retainer 8 abuts against the proximal end 60 of the needle shield 6.

Figure 6D schematically illustrates the anti-rattling member 91 pressing the retainer 8, the medical container 4 and the power pack 7 against the top body 30 to suppress the clearances and thus the rattling noise.

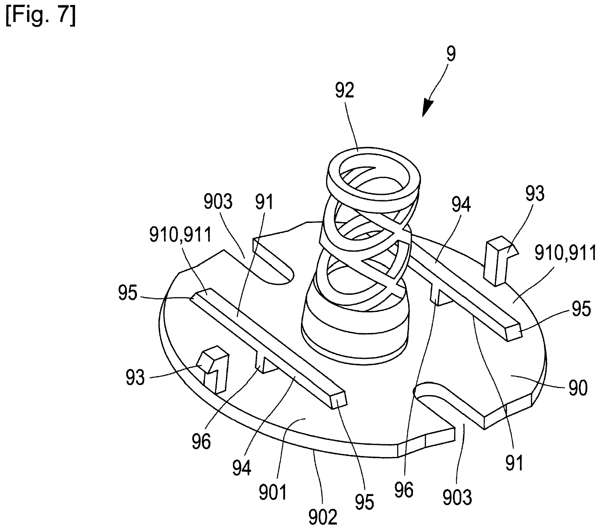

As illustrated in the embodiment of Figure 7, the anti-rattling member 91 may be a resiliently deformable member which may be in the form of a T-shaped blade. The T-shaped blade 91 has one transversal arm 94 extending orthogonal to the longitudinal axis A between two free ends 95. A connecting rod 96 extends in the axial direction for connecting the transversal arm 94 to the proximal side 901 of the plug 9. The T-shaped blade 91 has a proximal abutment surface 910 which may be defined by the upper surface of the transversal arm 94, opposite the connecting rod 96. The proximal abutment surface 910 is configured to abut against the distal end 84 of the retainer 8 to push said retainer 8 in the proximal direction. The plug 9 may include one, two or more T-shaped blades 91 which may be arranged diametrically opposite to each other. The T-shaped blades 91 are arranged at a distance from the lateral edge of the sealing portion 90. As illustrated in Figure 4, the connecting rod 96 may be arranged at the middle of the transversal arm 94.

As visible in Figure 7, the plug 9 may further include a push-back member 92 for automatically performing the push-back step, in the same way as the push-back member 92 of the plug 9 illustrated in Figure 6A. Thus, the push-back member 92 includes a resilient element, such as a spring, which may be arranged at the center of the the transversal plate of the sealing portion 90 and which is configured to face the distal end 61 of the needle shield 6. The resilient element 92 extends axially and is configured to automatically perform the push-back step, i.e. without need for a machine and an additional time-consuming step to push the retainer 8 in the proximal direction. The resilient element 92 may be arranged between the T-shaped bladed 91, at a distance from the T-shaped blades 91. The resilient element 92 is preferably integral with the plug 9, which may thus be made of a single piece.

It is to be understood that the present invention is not limited to the embodiments described above and illustrated in the drawings; rather, the skilled person will recognize that many changes and modifications may be made within the scope of the appended claims.

## Claims

1. Cap (5) for removing a needle shield (6) attached to a medical container (4) arranged inside a body (20) of an autoinjector (1), the cap (5) including:
a housing (50) extending along a longitudinal axis A, the housing (50) having a distal end (51) and an opposite proximal end configured to be removably attached to the body (20) of the autoinjector (1),
a retainer (8) arranged inside the housing (50) and axially movable with respect to said housing (50), the retainer (8) including an inner cavity (52) for receiving the needle shield (6), a clamping member (81) configured to remove the needle shield (6) from the medical container (4), an opened distal end (84) configured to provide access to a distal end (61) of the needle shield (6), and a proximal abutment surface (87) configured to abut against the medical container (4),
a plug (9) arranged at the distal end (51) of the housing (50), the plug (9) including a sealing portion (90) configured for closing the distal end (51) of the housing (50) and at least one anti-rattling member (91) arranged at a proximal side (901) of the sealing portion (90) for abutting against the retainer (8) and move the retainer (8) in the proximal direction.

2. Cap (5) according to the preceding claim, wherein the anti-rattling member (91) proximally protrudes from the proximal side (901) of the sealing portion (90).

3. Cap (5) according to any one of the preceding claims, wherein the plug (9) includes a single anti-rattling member (91).

4. Cap (5) according to any one of the preceding claims, wherein the anti-rattling member (91) includes a sloped surface (910) configured to deform a distally extending resilient leg of the retainer (8).

5. Cap (5) according to any one of the preceding claims, wherein the anti-rattling member (91) includes a resililently deformable element configured to exert a proximal pushing force on the retainer (8).

6. Cap (5) according to the preceding claim, wherein the resiliently deformable member is in the form of a T-shaped blade.

7. Cap (5) according to any one of the preceding claims, wherein the plug (9) further includes a push-back member (92) for allowing to push the needle shield (6) in a proximal direction.

8. Cap (5) according to the preceding claim, wherein the push-back member (92) is a through-hole (920) extending through the sealing portion (90) and configured to axially face a distal end (61) of the needle shield (6).

9. Cap (5) according to any one of the preceding claims 7 to 8, wherein the push-back member (92) includes a spring configured to push the needle shield (6) in the proximal direction.

10. Cap (5) according to any one of the preceding claims, wherein the sealing portion (90) is plate-shaped.

11. Cap (5) according to any one of the preceding claims, wherein the plug (9) includes securing means for securing the plug (9) to the housing (50).

12. Cap (5) according to any one of the preceding claims 1 to 10, wherein the plug (9) and the housing (50) are made of a single piece.

13. Cap (5) according to any one of the preceding claims, wherein the plug (9) is made of a single piece.

14. Autoinjector (1) including a cap (5) according to any one of the preceding claims.

15. Method for assembling the autoinjector (1) according to claim 14, the method including the steps of :
(i) attaching the plug (9) to the cap housing (50), said attachment causing the plug (9) to push the retainer (8) in the proximal direction so that the medical container (4) abuts against a power pack (7) of the autoinjector (1) and the power pack (7) abuts against the body of the autoinjector (1) ;
(ii) pushing the needle shield (6) back in the proximal direction so that the clamping member (81) of the retainer (8) engages an axial gap (10) between the needle shield (6) and a barrel (40) of the medical container (4).

## Patentansprüche

1. Kappe (5) zum Entfernen eines Nadelschutzes (6), der an einem medizinischen Behälter (4) befestigt ist, der innerhalb eines Körpers (20) eines Autoinjektors (1) angeordnet ist, wobei die Kappe (5) beinhaltet:
ein Gehäuse (50), das sich entlang einer Längsachse A erstreckt, wobei das Gehäuse (50) ein distales Ende (51) und ein gegenüberliegendes proximales Ende aufweist, und konfiguriert ist, um abnehmbar am Körper (20) des Autoinjektors (1) befestigt zu werden,
einen Halter (8), der innerhalb des Gehäuses (50) angeordnet ist, und in Bezug auf das Gehäuse (50) axial beweglich ist, wobei der Halter (8) einen inneren Hohlraum (52) zur Aufnahme des Nadelschutzes (6) beinhaltet, ein Klemmelement (81), das konfiguriert ist, um den Nadelschutz (6) von dem medizinischen Behälter (4) abzunehmen, ein geöffnetes distales Ende (84), das konfiguriert ist, um Zugriff auf ein distales Ende (61) des Nadelschutzes (6) bereitzustellen, und eine proximale Anlegeoberfläche (87), die konfiguriert ist, um sich an den medizinischen Behälter (4) anzulegen,
einen Stecker (9), der am distalen Ende (51) des Gehäuses (50) angeordnet ist, wobei der Stecker (9) einen Dichtungsabschnitt (90) beinhaltet, der zum Schließen des distalen Endes (51) des Gehäuses (50) konfiguriert ist, und mindestens ein Klapperschutzelement (91), das auf einer proximalen Seite (901) des Dichtungsabschnitts (90) zum Anlegen an den Halter (8) angeordnet ist, und um den Halter (8) in der proximalen Richtung zu bewegen.

2. Kappe (5) nach dem vorstehenden Anspruch, wobei das Klapperschutzelement (91) proximal aus der proximalen Seite (901) des Dichtungsabschnitts (90) hervorsteht.

3. Kappe (5) nach einem der vorstehenden Ansprüche, wobei der Stecker (9) ein einziges Klapperschutzelement (91) beinhaltet.

4. Kappe (5) nach einem der vorstehenden Ansprüche, wobei das Klapperschutzelement (91) eine geneigte Oberfläche (910) beinhaltet, die konfiguriert ist, um einen sich distal erstreckenden elastischen Schenkel des Halters (8) zu verformen.

5. Kappe (5) nach einem der vorstehenden Ansprüche, wobei das Klapperschutzelement (91) ein elastisch verformbares Element beinhaltet, das konfiguriert ist, um eine proximale Schubkraft auf den Halter (8) auszuüben.

6. Kappe (5) nach dem vorstehenden Anspruch, wobei das elastisch verformbare Element in Form einer T-förmigen Klinge ist.

7. Kappe (5) nach einem der vorstehenden Ansprüche, wobei der Stecker (9) weiter ein Rückschubelement (92) zum Ermöglichen beinhaltet, den Nadelschutz (6) in eine proximale Richtung zu schieben.

8. Kappe (5) nach dem vorstehenden Anspruch, wobei das Rückschubelement (92) ein Durchgangsloch (920) ist, das sich durch den Dichtungsabschnitt (90) hindurch erstreckt, und konfiguriert ist, um einem distalen Ende (61) des Nadelschutzes (6) axial zugewandt zu sein.

9. Kappe (5) nach einem der vorstehenden Ansprüche 7 bis 8, wobei das Rückschubelement (92) eine Feder beinhaltet, die konfiguriert ist, um den Nadelschutz (6) in die proximale Richtung zu schieben.

10. Kappe (5) nach einem der vorstehenden Ansprüche, wobei der Dichtungsabschnitt (90) plattenförmig ist.

11. Kappe (5) nach einem der vorstehenden Ansprüche, wobei der Stecker (9) Sicherungsmittel zum Sichern des Steckers (9) an dem Gehäuse (50) beinhaltet.

12. Kappe (5) nach einem der vorstehenden Ansprüche 1 bis 10, wobei der Stecker (9) und das Gehäuse (10) aus einem einzigen Stück gefertigt sind.

13. Kappe (5) nach einem der vorstehenden Ansprüche, wobei der Stecker (9) aus einem einzigen Stück gefertigt ist.

14. Autoinjektor (1), der eine Kappe (5) nach einem der vorstehenden Ansprüche beinhaltet.

15. Verfahren zum Zusammensetzen eines Autoinjektors (1) nach Anspruch 14, wobei das Verfahren die Schritte beinhaltet zum:
(i) Befestigen des Steckers (9) am Kappengehäuse (50), wobei das Befestigen den Stecker (9) veranlasst, den Halter (8) in die proximale Richtung zu schieben, sodass sich der medizinische Behälter (4) an ein Netzteil (7) des Autoinjektors (1) anlegt, und sich das Netzteil (7) an den Körper des Autoinjektors (1) anlegt;
(ii) Schieben des Nadelschutzes (6) zurück in die proximale Richtung, sodass das Klemmelement (81) des Halters (8) in einen axialen Spalt (10) zwischen dem Nadelschutz (6) und einem Schaft (40) des medizinischen Behälters (4) eingreift.

## Revendications

1. Capuchon (5) pour retirer une protection d'aiguille (6) fixée à un récipient médical (4) agencé à l'intérieur d'un corps (20) d'un auto-injecteur (1), le capuchon (5) comprenant :
un boîtier (50) s'étendant le long d'un axe longitudinal A, le boîtier (50) ayant une extrémité distale (51) et une extrémité proximale opposée configurée pour être fixée de manière amovible au corps (20) de l'auto-injecteur (1),
un dispositif de retenue (8) agencé à l'intérieur du boîtier (50) et mobile axialement par rapport audit boîtier (50), le dispositif de retenue (8) comprenant une cavité interne (52) pour recevoir la protection d'aiguille (6), un organe de serrage (81) configuré pour retirer la protection d'aiguille (6) du récipient médical (4), une extrémité distale ouverte (84) configurée pour donner accès à une extrémité distale (61) de la protection d'aiguille (6), et une surface de butée proximale (87) configurée pour venir en butée contre le récipient médical (4),
un bouchon (9) agencé au niveau de l'extrémité distale (51) du boîtier (50), le bouchon (9) comprenant une partie d'étanchéité (90) configurée pour fermer l'extrémité distale (51) du boîtier (50) et au moins un organe anti-cliquetis (91) agencé sur un côté proximal (901) de la partie d'étanchéité (90) pour venir en butée contre le dispositif de retenue (8) et déplacer le dispositif de retenue (8) dans la direction proximale.

2. Capuchon (5) selon la revendication précédente, dans lequel l'organe anti-cliquetis (91) fait saillie de manière proximale depuis le côté proximal (901) de la partie d'étanchéité (90).

3. Capuchon (5) selon l'une quelconque des revendications précédentes, dans lequel le bouchon (9) comprend un seul organe anti-cliquetis (91).

4. Capuchon (5) selon l'une quelconque des revendications précédentes, dans lequel l'organe anti-cliquetis (91) comprend une surface inclinée (910) configurée pour déformer une patte élastique s'étendant de manière distale du dispositif de retenue (8).

5. Capuchon (5) selon l'une quelconque des revendications précédentes, dans lequel l'organe anti-cliquetis (91) comprend un élément élastiquement déformable configuré pour exercer une force de poussée proximale sur le dispositif de retenue (8).

6. Capuchon (5) selon la revendication précédente, dans lequel l'organe élastiquement déformable se présente sous la forme d'une lame en forme de T.

7. Capuchon (5) selon l'une quelconque des revendications précédentes, dans lequel le bouchon (9) comprend en outre un organe de refoulement (92) pour permettre de pousser la protection d'aiguille (6) dans une direction proximale.

8. Capuchon (5) selon la revendication précédente, dans lequel l'organe de refoulement (92) est un trou traversant (920) s'étendant à travers la partie d'étanchéité (90) et configuré pour faire face axialement à une extrémité distale (61) de la protection d'aiguille (6).

9. Capuchon (5) selon l'une quelconque des revendications précédentes 7 et 8, dans lequel l'organe de refoulement (92) comprend un ressort configuré pour pousser la protection d'aiguille (6) dans la direction proximale.

10. Capuchon (5) selon l'une quelconque des revendications précédentes, dans lequel la partie d'étanchéité (90) est en forme de plaque.

11. Capuchon (5) selon l'une quelconque des revendications précédentes, dans lequel le bouchon (9) comprend des moyens de fixation pour fixer le bouchon (9) au boîtier (50).

12. Capuchon (5) selon l'une quelconque des revendications précédentes 1 à 10, dans lequel le bouchon (9) et le boîtier (50) sont réalisés en une seule pièce.

13. Capuchon (5) selon l'une quelconque des revendications précédentes, dans lequel le bouchon (9) est réalisé en une seule pièce.

14. Auto-injecteur (1) comprenant un capuchon (5) selon l'une quelconque des revendications précédentes.

15. Procédé d'assemblage de l'auto-injecteur (1) selon la revendication 14, le procédé comprenant les étapes :
(i) de fixation du bouchon (9) au boîtier de capuchon (50), ladite fixation amenant le bouchon (9) à pousser le dispositif de retenue (8) dans la direction proximale de sorte que le récipient médical (4) vienne en butée contre un bloc d'alimentation (7) de l'auto-injecteur (1) et que le bloc d'alimentation (7) vienne en butée contre le corps de l'auto-injecteur (1) ;
(ii) de refoulement de la protection d'aiguille (6) dans la direction proximale de sorte que l'organe de serrage (81) du dispositif de retenue (8) s'engage dans un espace axial (10) entre la protection d'aiguille (6) et un cylindre (40) du récipient médical (4).
